# EUROPEAN PATENT APPLICATION

(11) **EP 4 733 318 A2**
(43) Date of publication of application: **29.04.2026**
(21) Application number: 25211165.3
(22) Date of filing: 24.10.2025
(51) Int. Cl.: C07K 16/114, G01N 33/569, A61K 39/00

(54) **ANTIBODY AGAINST HUMAN IMMUNODEFICIENCY VIRUS TYPE 1 P24 AND TYPE 2 P26 AND USES THEREOF**

(30) Priority: 25.10.2024 CN 202411507204
(71) Applicant: Shenzhen Mindray Bio-Medical Electronics Co., Ltd., Shenzhen, Guangdong 518057 (CN); Hytest Ltd., 20520 Turku (FI)
(72) Inventor: ZHAN, Chengxiong, Shenzhen, 518057 (CN); HAN, Qiang, Shenzhen, 518057 (CN); XIE, Xiaqing, Shenzhen, 518057 (CN); TANG, Junhui, Shenzhen, 518057 (CN); TAMM, Natalia N., 117186 Moscow (RU); MIKHAILUK, Evgenia A., 117186 Moscow (RU); POPOVA, Anfisa S., 117186 Moscow (RU); KOGAN, Alexander E., 117186 Moscow (RU); SOKOLOVA, Elizaveta E., 117186 Moscow (RU); KOZLOVSKY, Stanislav V., 117186 Moscow (RU); GRUZDEVA, Natalia A., 117186 Moscow (RU); TRUFANOVA, Anna A., 117186 Moscow (RU); MISHIN, Alexander S., 117186 Moscow (RU); ROZOV, Fedor N., 117186 Moscow (RU); KOTLOV, Sergei A., 117186 Moscow (RU); GAINOVA, Kristina M., 117186 Moscow (RU); POSTNIKOV, Alexander B., 117186 Moscow (RU); KATRUKHA, Alexey G., 117186 Moscow (RU)
(74) Representative: KIPA AB

(57) **Abstract**

The present disclosure relates to the field of biomedical technology, and specifically to an antibody against human immunodeficiency virus type 1 p24 and type 2 p26 and uses thereof. The present disclosure provides an antibody or antigen-binding fragment thereof capable of specifically binding to human immunodeficiency virus type 1 p24 protein and human immunodeficiency virus type 2 p26 protein. The present disclosure also provides an antibody pair, kit and related uses thereof. Utilizing the antibody pair in immunoassays enables the simultaneous detection of HIV-1 p24 and HIV-2 p26 antigens, with the advantage of a lower limit of detection (LOD).

## Description

### CROSS REFERENCE

This application claims benefit of priority of Chinese Patent Application No. 202411507204.4 filed on October 25, 2024, the content of which is incorporated herein by reference in its entirety.

### TECHNICAL FIELD

The present disclosure relates to the field of biomedical technology, and specifically to an antibody against human immunodeficiency virus type 1 p24 and type 2 p26 and uses thereof.

### BACKGROUND ART

Human immunodeficiency virus (HIV) is a human retrovirus that causes a series of diseases such as acquired immunodeficiency syndrome (AIDS) and AIDS-related syndrome (ARC). Currently, there is a lack of effective vaccines and effective treatments for these diseases. Therefore, early diagnosis of HIV through effective HIV-specific detection methods is of great importance.

There are two types of HIV, i.e., HIV-1 and HIV-2. Although they share similarities across 15 genes/proteins with the exception of one gene/protein each, namely VPU in HIV-1 and VPX in HIV-2, they exhibit only 40-60% homology at the amino acid sequence level, particularly in their envelope proteins. HIV-1 and HIV-2 have different infectivity, pathogenicity and epidemic intensity. Currently, HIV-1 causes widespread AIDS worldwide, whereas HIV-2 mainly occurs in West African countries and has shown a tendency to spread globally in recent years. In recent years, infections caused by HIV-2 in China have become common.

Therefore, those skilled in the art hope to develop an immunoassay method that can simultaneously detect HIV-1 and HIV-2.

### CONTENTS OF THE PRESENT DISCLOSURE

In view of this, the present disclosure discloses an antibody against human immunodeficiency virus type 1 p24 and type 2 p26 and uses thereof.

In the first aspect of the present disclosure, there is provided an antibody or antigen-binding fragment thereof capable of specifically binding to human immunodeficiency virus type 1 p24 protein and human immunodeficiency virus type 2 p26 protein, which comprises a heavy chain variable region comprising HCDR1, HCDR2 and HCDR3 and a light chain variable region comprising LCDR1, LCDR2 and LCDR3;
wherein, the HCDR1 has an amino acid sequence selected from SEQ ID NOs: 1, 7, 13, 19, 25, 31, 37 or variants thereof; the HCDR2 has an amino acid sequence selected from SEQ ID NOs: 2, 8, 14, 20, 26, 32, 38 or variants thereof; the HCDR3 has an amino acid sequence selected from SEQ ID NOs: 3, 9, 15, 21, 27, 33, 39 or variants thereof;
the LCDR1 has an amino acid sequence selected from SEQ ID NOs: 4, 10, 16, 22, 28, 34, 40 or variants thereof; the LCDR2 has an amino acid sequence selected from SEQ ID NOs: 5, 11, 17, 23, 29, 35, 41 or variants thereof; the LCDR3 has an amino acid sequence selected from SEQ ID NOs: 6, 12, 18, 24, 30, 36, 42 or variants thereof;
wherein the variant comprises an amino acid mutation as compared with the amino acid sequence from which it is derived, and the amino acid mutation is a substitution, deletion or addition of one or several amino acids (e.g., a substitution, deletion or addition of 1, 2, 3, 4 or 5 amino acids); the variant has an identity of at least 80%, at least 85%, at least 90%, at least 91%, at least 92%, at least 93%, at least 94%, at least 95%, at least 96%, at least 97%, at least 98% or at least 99% as compared with the amino acid sequence from which it is derived.

In some embodiments, the substitution is a conservative substitution.

In some embodiments, the amino acid sequences of HCDR1, HCDR2 and HCDR3 are selected from one of the following groups:
the amino acid sequence of HCDR1 is SEQ ID NO: 1, the amino acid sequence of HCDR2 is SEQ ID NO: 2, and the amino acid sequence of HCDR3 is SEQ ID NO: 3;
the amino acid sequence of HCDR1 is SEQ ID NO: 7, the amino acid sequence of HCDR2 is SEQ ID NO: 8, and the amino acid sequence of HCDR3 is SEQ ID NO: 9;
the amino acid sequence of HCDR1 is SEQ ID NO: 13, the amino acid sequence of HCDR2 is SEQ ID NO: 14, and the amino acid sequence of HCDR3 is SEQ ID NO: 15;
the amino acid sequence of HCDR1 is SEQ ID NO: 19, the amino acid sequence of HCDR2 is SEQ ID NO: 20, and the amino acid sequence of HCDR3 is SEQ ID NO: 21;
the amino acid sequence of HCDR1 is SEQ ID NO: 25, the amino acid sequence of HCDR2 is SEQ ID NO: 26, and the amino acid sequence of HCDR3 is SEQ ID NO: 27;
the amino acid sequence of HCDR1 is SEQ ID NO: 31, the amino acid sequence of HCDR2 is SEQ ID NO: 32, and the amino acid sequence of HCDR3 is SEQ ID NO: 33; or,
the amino acid sequence of HCDR1 is SEQ ID NO: 37, the amino acid sequence of HCDR2 is SEQ ID NO: 38, and the amino acid sequence of HCDR3 is SEQ ID NO: 39;
the amino acid sequences of LCDR1, LCDR2 and LCDR3 are selected from one of the following groups:
   the amino acid sequence of LCDR1 is SEQ ID NO: 4, the amino acid sequence of LCDR2 is SEQ ID NO: 5, and the amino acid sequence of LCDR3 is SEQ ID NO: 6;
   the amino acid sequence of LCDR1 is SEQ ID NO: 10, the amino acid sequence of LCDR2 is SEQ ID NO: 11, and the amino acid sequence of LCDR3 is SEQ ID NO: 12;
   the amino acid sequence of LCDR1 is SEQ ID NO: 16, the amino acid sequence of LCDR2 is SEQ ID NO: 17, and the amino acid sequence of LCDR3 is SEQ ID NO: 18;
   the amino acid sequence of LCDR1 is SEQ ID NO: 22, the amino acid sequence of LCDR2 is SEQ ID NO: 23, and the amino acid sequence of LCDR3 is SEQ ID NO: 24;
   the amino acid sequence of LCDR1 is SEQ ID NO: 28, the amino acid sequence of LCDR2 is SEQ ID NO: 29, and the amino acid sequence of LCDR3 is SEQ ID NO: 30;
   the amino acid sequence of LCDR1 is SEQ ID NO: 34, the amino acid sequence of LCDR2 is SEQ ID NO: 35, and the amino acid sequence of LCDR3 is SEQ ID NO: 36; or,
   the amino acid sequence of LCDR1 is SEQ ID NO: 40, the amino acid sequence of LCDR2 is SEQ ID NO: 41, and the amino acid sequence of LCDR3 is SEQ ID NO: 42.

In some embodiments, the antibody or antigen-binding fragment thereof comprises a heavy chain variable region comprising HCDR1, HCDR2 and HCDR3 and a light chain variable region comprising LCDR1, LCDR2 and LCDR3;
wherein the amino acid sequence of HCDR1 is SEQ ID NO: 1, the amino acid sequence of HCDR2 is SEQ ID NO: 2, and the amino acid sequence of HCDR3 is SEQ ID NO: 3; the amino acid sequence of LCDR1 is SEQ ID NO: 4, the amino acid sequence of LCDR2 is SEQ ID NO: 5, and the amino acid sequence of LCDR3 is SEQ ID NO: 6; or,
the amino acid sequence of HCDR1 is SEQ ID NO: 7, the amino acid sequence of HCDR2 is SEQ ID NO: 8, and the amino acid sequence of HCDR3 is SEQ ID NO: 9; the amino acid sequence of LCDR1 is SEQ ID NO: 10, the amino acid sequence of LCDR2 is SEQ ID NO: 11, and the amino acid sequence of LCDR3 is SEQ ID NO: 12; or,
the amino acid sequence of HCDR1 is SEQ ID NO: 13, the amino acid sequence of HCDR2 is SEQ ID NO: 14, the amino acid sequence of HCDR3 is SEQ ID NO: 15; the amino acid sequence of LCDR1 is SEQ ID NO: 16, the amino acid sequence of LCDR2 is SEQ ID NO: 17, and the amino acid sequence of LCDR3 is SEQ ID NO: 18; or,
the amino acid sequence of HCDR1 is SEQ ID NO: 19, the amino acid sequence of HCDR2 is SEQ ID NO: 20, and the amino acid sequence of HCDR3 is SEQ ID NO: 21; the amino acid sequence of LCDR1 is SEQ ID NO: 22, the amino acid sequence of LCDR2 is SEQ ID NO: 23, and the amino acid sequence of LCDR3 is SEQ ID NO: 24; or,
the amino acid sequence of HCDR1 is SEQ ID NO: 25, the amino acid sequence of HCDR2 is SEQ ID NO: 26, and the amino acid sequence of HCDR3 is SEQ ID NO: 27; the amino acid sequence of LCDR1 is SEQ ID NO: 28, the amino acid sequence of LCDR2 is SEQ ID NO: 29, and the amino acid sequence of LCDR3 is SEQ ID NO: 30; or,
the amino acid sequence of HCDR1 is SEQ ID NO: 31, the amino acid sequence of HCDR2 is SEQ ID NO: 32, and the amino acid sequence of HCDR3 is SEQ ID NO: 33; the amino acid sequence of LCDR1 is SEQ ID NO: 34, the amino acid sequence of LCDR2 is SEQ ID NO: 35, and the amino acid sequence of LCDR3 is SEQ ID NO: 36; or,
the amino acid sequence of HCDR1 is SEQ ID NO: 37, the amino acid sequence of HCDR2 is SEQ ID NO: 38, and the amino acid sequence of HCDR3 is SEQ ID NO: 39; the amino acid sequence of LCDR1 is SEQ ID NO: 40, the amino acid sequence of LCDR2 is SEQ ID NO: 41, and the amino acid sequence of LCDR3 is SEQ ID NO: 42.

In some embodiments, the antibody is a full-length antibody, a Fab fragment, a Fab' fragment, a F(ab')₂ fragment, a double-chain Fv fragment, or a single-chain Fv fragment.

In some embodiments, the antibody is a monoclonal antibody.

In some embodiments, the type of the antibody or antigen-binding fragment thereof is IgG, IgM, IgE, IgD or IgA.

In some embodiments, the antibody is a mouse antibody, a rabbit antibody or a humanized antibody.

In some embodiments, the antibody comprises a constant region.

In some embodiments, the constant region comprises a heavy chain constant region and/or a light chain constant region.

In some embodiments, the constant region comprises a mouse constant region, a rat constant region, a rabbit constant region, a sheep constant region, a monkey constant region, or a human constant region.

In some embodiments, the heavy chain constant region is a heavy chain constant region of IgG1, IgG2, IgG3 or IgG4.

In some embodiments, the light chain constant region is a κ or λ type light chain constant region.

In some embodiments, the antibody or antigen-binding fragment thereof is also coupled with a detection label; the detection label comprises at least one selected from the group consisting of a radioactive label, a fluorescent label, a chemiluminescent label, a biotin, a colloidal gold, an electrochemiluminescent label, a quantum dot or an enzyme.

In some embodiments, the antibody or antigen-binding fragment thereof is produced by a hybridoma cell line deposited in VKPM with a deposit number of VKPM H-227.

In some embodiments, the antibody or antigen-binding fragment thereof is produced by a hybridoma cell line deposited in VKPM with a deposit number of VKPM H-228.

In some embodiments, the antibody or antigen-binding fragment thereof is produced by a plasmid in *Escherichia coli* deposited in VKPM with a deposit number of VKPM B-15024 and a plasmid in *Escherichia coli* deposited in VKPM with a deposit number of VKPM B-15025.

In some embodiments, the antibody or antigen-binding fragment thereof is produced by a plasmid in *Escherichia coli* deposited in VKPM with a deposit number of VKPM B-15026 and a plasmid in *Escherichia coli* deposited in VKPM with a deposit number of VKPM B-15027.

In some embodiments, the antibody or antigen-binding fragment thereof is produced by a plasmid in *Escherichia coli* deposited in VKPM with a deposit number of VKPM B-15028 and a plasmid in *Escherichia coli* deposited in VKPM with a deposit number of VKPM B-15029.

In some embodiments, the antibody or antigen-binding fragment thereof is produced by a plasmid in *Escherichia coli* deposited in VKPM with a deposit number of VKPM B-15030 and a plasmid in *Escherichia coli* deposited in VKPM with a deposit number of VKPM B-15031.

In some embodiments, the antibody or antigen-binding fragment thereof is produced by a plasmid in *Escherichia coli* deposited in VKPM with a deposit number of VKPM B-15032 and a plasmid in *Escherichia coli* deposited in VKPM with a deposit number of VKPM B-15033.

The second aspect of the present disclosure provides an isolated polynucleotide, which encodes the antibody or antigen-binding fragment thereof described in the first aspect of the present disclosure.

The third aspect of the present disclosure provides a vector, which comprises the isolated polynucleotide described in the second aspect of the present disclosure.

The fourth aspect of the present disclosure provides a host cell, which comprises the isolated polynucleotide described in the second aspect of the present disclosure, or the vector described in the third aspect of the present disclosure.

The fifth aspect of the present disclosure provides an antibody pair, which comprises a capture antibody and a detection antibody, and at least one of the capture antibody and the detection antibody is selected from the antibody or antigen-binding fragment thereof described in the first aspect of the present disclosure.

In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase.

In some embodiments, the capture antibody is coated on the surface of a solid carrier, e.g., magnetic bead or microtiter plate. In some embodiments, the capture antibody is coated on the surface of a magnetic bead.

In some embodiments, the capture antibody and the detection antibody are each independently selected from the antibody or antigen-binding fragment thereof described in the first aspect of the present disclosure, and the capture antibody and the detection antibody are different.

In some embodiments, the antibody pair is selected from any one of the following groups (i) to (vi):
(i) a capture antibody, which comprises a HCDR1 with an amino acid sequence as set forth in SEQ ID NO: 7, a HCDR2 with an amino acid sequence as set forth in SEQ ID NO: 8, a HCDR3 with an amino acid sequence as set forth in SEQ ID NO: 9, a LCDR1 with an amino acid sequence as set forth in SEQ ID NO: 10, a LCDR2 with an amino acid sequence as set forth in SEQ ID NO: 11, and a LCDR3 with an amino acid sequence as set forth in SEQ ID NO: 12; in some embodiments, the capture antibody is p24_138; and,
   a detection antibody, which is one selected from the following group:
   the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42; in some embodiments, the detection antibody is p24_rec_252;
   the detection antibody comprising a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, and a LCDR3 with an amino acid sequence as set forth in SEQ ID NO: 6; in some embodiments, the detection antibody is p24_113;
   the detection antibody comprising a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33, a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; in some embodiments, the detection antibody is p24_rec_179;
   the detection antibody comprising a HCDR1 with an amino acid sequence as set forth in SEQ ID NO: 25, a HCER2 with an amino acid sequence as set forth in SEQ ID NO: 26, a HCER3 with an amino acid sequence as set forth in SEQ ID NO: 27, a LCDR1 with an amino acid sequence as set forth in SEQ ID NO: 28, a LCDR2 with an amino acid sequence as set forth in SEQ ID NO: 29, a LCDR3 with an amino acid sequence as set forth in SEQ ID NO: 30; in some embodiments, the detection antibody is p24_rec_151;
   the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, a LCDR3 with an amino acid sequence as set forth in SEQ ID NO: 18; in some embodiments, the detection antibody is p24_rec_131; or,
   the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, a LCDR3 with an amino acid sequence as set forth in SEQ ID NO: 24; in some embodiments, the detection antibody is p24_rec_140;
(ii) a capture antibody, which comprises a HCDR1 with an amino acid sequence as set forth in SEQ ID NO: 25, a HCDR2 with an amino acid sequence as set forth in SEQ ID NO: 26, a HCDR3 with an amino acid sequence as set forth in SEQ ID NO: 27, a LCDR1 with an amino acid sequence as set forth in SEQ ID NO: 28, a LCDR2 with an amino acid sequence as set forth in SEQ ID NO: 29, and a LCDR3 with an amino acid sequence as set forth in SEQ ID NO: 30; in some embodiments, the capture antibody is p24_rec_151; and,
   a detection antibody, which is one selected from the following group:
   the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42; in some embodiments, the detection antibody is p24_rec_252;
   the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, and a LCDR3 with an amino acid sequence as set forth in SEQ ID NO: 24; in some embodiments, the detection antibody is p24_rec_140;
   the detection antibody comprising a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33, a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; in some embodiments, the detection antibody is p24_rec_179; or,
   the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, and a LCDR3 with an amino acid sequence as set forth in SEQ ID NO: 18; in some embodiments, the detection antibody is p24_rec_131;
(iii) a capture antibody, which comprises a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33, a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; in some embodiments, the capture antibody is p24_rec_179; and,
   a detection antibody, which is one selected from the following group:
   the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42; in some embodiments, the detection antibody is p24_rec_252; or,
   the detection antibody comprising a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, and a LCDR3 with an amino acid sequence as set forth in SEQ ID NO: 6; in some embodiments, the detection antibody is p24_113;
(iv) a capture antibody, which comprises a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, and a LCDR3 with an amino acid sequence as set forth in SEQ ID NO: 24; in some embodiments, the capture antibody is p24_rec_140; and,
   a detection antibody, which comprises a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42; in some embodiments, the detection antibody is p24_rec_252;
(v) a capture antibody, which comprises a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, and a LCDR3 with an amino acid sequence as set forth in SEQ ID NO: 18; in some embodiments, the capture antibody is p24_rec_131; and,
   a detection antibody, which comprises a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42; in some embodiments, the detection antibody is p24_rec_252;
(vi) a capture antibody, which comprises a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, and a LCDR3 with an amino acid sequence as set forth in SEQ ID NO: 6; in some embodiments, the capture antibody is p24_113; and,
   a detection antibody, which is one selected from the following group:
   the detection antibody comprising a HCDR1 with an amino acid sequence as set forth in SEQ ID NO: 25, a HCDR2 with an amino acid sequence as set forth in SEQ ID NO: 26, a HCDR3 with an amino acid sequence as set forth in SEQ ID NO: 27, a LCDR1 with an amino acid sequence as set forth in SEQ ID NO: 28, a LCDR2 with an amino acid sequence as set forth in SEQ ID NO: 29, and a LCDR3 with an amino acid sequence as set forth in SEQ ID NO: 30; in some embodiments, the detection antibody is p24_rec_151;
   the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, and a LCDR3 with an amino acid sequence as set forth in SEQ ID NO: 18; in some embodiments, the detection antibody is p24_rec_131; or,
   the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, and a LCDR3 with an amino acid sequence as set forth in SEQ ID NO: 24; in some embodiments, the detection antibody is p24_rec_140.

In some embodiments, the antibody pair is any one selected from the following groups (a) to (f):
(a) a capture antibody, which is produced by a hybridoma cell line deposited in VKPM with a deposit number of VKPM H-227; in some embodiments, the capture antibody is p24_138; and,
   a detection antibody, which is one selected from the following group:
   the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033; in some embodiments, the detection antibody is p24_rec_252;
   the detection antibody is an antibody produced by a hybridoma cell line deposited in VKPM with a deposit number of VKPM H-228; in some embodiments, the detection antibody is p24_113;
   the detection antibody is an antibody produced by a plasmid in *Escherichia coli* deposited in VKPM with a deposit number of VKPM B-15030 and a plasmid in *Escherichia coli* deposited in VKPM with a deposit number of VKPM B-15031; in some embodiments, the detection antibody is p24_rec_179;
   the detection antibody is an antibody produced by a plasmid in *Escherichia coli* deposited in VKPM with a deposit number of VKPM B-15028 and a plasmid in *Escherichia coli* deposited in VKPM with a deposit number of VKPM B-15029; in some embodiments, the detection antibody is p24_rec_151;
   the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025; in some embodiments, the detection antibody is p24_rec_131; or,
   the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027; in some embodiments, the detection antibody is p24_rec_140;
(b) a capture antibody, which is an antibody produced by a plasmid in *Escherichia coli* deposited in VKPM with a deposit number of VKPM B-15028 and a plasmid in *Escherichia coli* deposited in VKPM with a deposit number of VKPM B-15029; in some embodiments, the capture antibody is p24_rec_151; and,
   a detection antibody, which is one selected from the following group:
   the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033; in some embodiments, the detection antibody is p24_rec_252;
   the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027; in some embodiments, the detection antibody is p24_rec_140;
   the detection antibody is an antibody produced by a plasmid in *Escherichia coli* deposited in VKPM with a deposit number of VKPM B-15030 and a plasmid in *Escherichia coli* deposited in VKPM with a deposit number of VKPM B-15031; in some embodiments, the detection antibody is p24_rec_179; or,
   the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025; in some embodiments, the detection antibody is p24_rec_131;
(c) a capture antibody, which is an antibody produced by a plasmid in *Escherichia coli* deposited in VKPM with a deposit number of VKPM B-15030 and a plasmid in *Escherichia coli* deposited in VKPM with a deposit number of VKPM B-15031; in some embodiments, the capture antibody is p24_rec_179; and,
   a detection antibody, which is one selected from the following group:
   the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033; in some embodiments, the detection antibody is p24_rec_252; or,
   the detection antibody is an antibody produced by a hybridoma cell line deposited in VKPM with a deposit number of VKPM H-228; in some embodiments, the detection antibody is p24_113;
(d) a capture antibody, which is an antibody produced by a plasmid in *Escherichia coli* deposited in VKPM with a deposit number of VKPM B-15026 and a plasmid in *Escherichia coli* deposited in VKPM with a deposit number of VKPM B-15027; in some embodiments, the capture antibody is p24_rec_140; and,
   a detection antibody, which is an antibody produced by a plasmid in *Escherichia coli* deposited in VKPM with a deposit number of VKPM B-15032 and a plasmid in *Escherichia coli* deposited in VKPM with a deposit number of VKPM B-15033; in some embodiments, the detection antibody is p24_rec_252;
(e) a capture antibody, which is an antibody produced by a plasmid in *Escherichia coli* deposited in VKPM with a deposit number of VKPM B-15024 and a plasmid in *Escherichia coli* deposited in VKPM with a deposit number of VKPM B-15025; in some embodiments, the capture antibody is p24_rec_131; and,
   a detection antibody, which is an antibody produced by a plasmid in *Escherichia coli* deposited in VKPM with a deposit number of VKPM B-15032 and a plasmid in *Escherichia coli* deposited in VKPM with a deposit number of VKPM B-15033; in some embodiments, the detection antibody is p24_rec_252;
(f) a capture antibody, which is an antibody produced by a hybridoma cell line deposited in VKPM with a deposit number of VKPM H-228; in some embodiments, the capture antibody is p24_113; and,
   a detection antibody, which is one selected from the following group:
   the detection antibody is an antibody produced by a plasmid in *Escherichia coli* deposited in VKPM with a deposit number of VKPM B-15028 and a plasmid in *Escherichia coli* deposited in VKPM with a deposit number of VKPM B-15029; in some embodiments, the detection antibody is p24_rec_151;
   the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025; in some embodiments, the detection antibody is p24_rec_131; or,
   the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027; in some embodiments, the detection antibody is p24_rec_140.

The sixth aspect of the present disclosure provides a kit, which comprises the antibody or antigen-binding fragment thereof described in the first aspect of the present disclosure, and the antibody pair described in the fifth aspect of the present disclosure.

The seventh aspect of the present disclosure provides use of the antibody or antigen-binding fragment thereof described in the first aspect of the present disclosure and the antibody pair described in the fifth aspect of the present disclosure in the manufacture of a product for detecting human immunodeficiency virus.

In some embodiments, the human immunodeficiency virus comprises human immunodeficiency virus type 1 and human immunodeficiency virus type 2.

The eighth aspect of the present disclosure provides a use of the antibody or antigen-binding fragment thereof described in the first aspect of the present disclosure and the antibody pair described in the fifth aspect of the present disclosure in the manufacture of a product for assisting the diagnosis of acquired immunodeficiency syndrome (AIDS) and AIDS-related syndrome (ARC).

The ninth aspect of the present disclosure provides a method for detecting presence and/or amount of human immunodeficiency virus or an antigen thereof in a sample, comprising: using the antibody or antigen-binding fragment thereof described in the first aspect of the present disclosure or the antibody pair described in the fifth aspect of the present disclosure to perform immunoassay on the sample.

In some embodiments, the method comprises:
(i) contacting the sample with the capture antibody of the antibody pair described in the fifth aspect of the present disclosure to form an antibody-antigen complex;
(ii) contacting the antibody-antigen complex with the detection antibody of the antibody pair to form an antibody-antigen-antibody complex; and
(iii) detecting the antibody-antigen-antibody complex, preferably wherein presence of the complex indicating presence of human immunodeficiency virus or an antigen thereof in the sample.

In some embodiments, the method is performed in vitro.

In some embodiments, the human immunodeficiency virus comprises human immunodeficiency virus type 1 and human immunodeficiency virus type 2. In some embodiments, the antigen is selected from HIV-1 antigen (e.g., p24) and HIV-2 antigen (e.g., p26).

In some embodiments, the immunoassay is selected from enzyme immunoassay or chemiluminescence immunoassay. In some embodiments, the detection antibody bears a detection label, e.g., enzyme, such as alkaline phosphatase. In some embodiments, the capture antibody is coated on the surface of a solid carrier, e.g., magnetic bead.

The tenth aspect of the present disclosure provides a method for diagnosing or aiding in the diagnosis of acquired immunodeficiency syndrome or AIDS-related syndrome, the method comprising: detecting the presence of human immunodeficiency virus or an antigen thereof in a sample from the subject by the method of ninth aspect; preferably wherein presence of human immunodeficiency virus or an antigen thereof indicating that the subject has sustained or may have sustained acquired immunodeficiency syndrome.

The above description is only an overview of the technical solution of the present application. In order to understand the technical means of the present application more clearly, it can be implemented according to the content of the description, and in order to make the above and other purposes, features and advantages of the present application more apparent and understandable, the specific embodiments of the present application are listed below.

### BRIEF DESCRIPTION OF THE DRAWINGS

Various other advantages and benefits of the present disclosure will become clear to one of ordinary skill in the art by reading the detailed description of the preferred embodiments below. The drawings are only used for the purpose of illustrating the preferred embodiments and are not considered to be limitations of the present disclosure. In the accompanying drawings:
FIG. 1 shows the SEC chromatographic analysis results of antibody p24_113;
FIG. 2 shows the SEC chromatographic analysis results of antibody p24_138;
FIG. 3 shows the SEC chromatographic analysis results of antibody P24_rec_131;
FIG. 4 shows the SEC chromatographic analysis results of antibody p24_rec_140;
FIG. 5 shows the SEC chromatographic analysis results of antibody p24_rec_151;
FIG. 6 shows the SEC chromatographic analysis results of antibody p24_rec_179;
FIG. 7 shows the SEC chromatographic analysis results of antibody p24_rec_252.

### EXEMPLARY EMBODIMENTS

Exemplary embodiments of the present disclosure will be described in more detail below with reference to the accompanying drawings. Although exemplary embodiments of the present disclosure are shown in the accompanying drawings, one of ordinary skill in the art will understand that the present disclosure can be implemented in various forms and should not be limited by the embodiments described herein. On the contrary, these embodiments are provided to enable a more thorough understanding of the present disclosure and to fully convey the scope of the present disclosure to one of ordinary skill in the art.

In the present disclosure, unless otherwise specified, the scientific and technical terms used herein have the meanings commonly understood by one of ordinary skill in the art. In addition, the cell culture, molecular genetics, nucleic acid chemistry, and immunology laboratory operation steps used herein are all routine steps widely used in corresponding fields. At the same time, in order to understand the present disclosure better, the definitions and explanations of the relevant terms are provided below.

As used herein, the term "antibody" refers to an immunoglobulin molecule that can specifically bind to a target via at least one antigen recognition site located in the variable region of the immunoglobulin molecule. The "antibody" used herein includes not only complete (i.e., full-length) antibodies, but also antigen-binding fragments thereof (e.g., Fab, Fab', F(ab')₂, Fv, scFv, Fd, CDR fragments), variants thereof, fusion proteins comprising antibodies, humanized antibodies, chimeric antibodies, double antibodies, linear antibodies, single-chain antibodies, multispecific antibodies (e.g., bispecific antibodies), and any other modified configurations of immunoglobulin molecules containing antigen recognition sites of desired specificity, including antibody glycosylation variants, antibody amino acid sequence variants, and covalently modified antibodies. Antibodies can be derived from any mammals, including but not limited to humans, monkeys, pigs, horses, rabbits, dogs, cats, rats, mice, etc., or other animals such as birds (e.g., chickens), fish (e.g., sharks), and camels (e.g., llamas).

Typically, a complete or full-length antibody contains two heavy chains and two light chains. Each heavy chain contains a heavy chain variable region (VH) and a heavy chain constant region (CH); each light chain contains a light chain variable region (VL) and a light chain constant region (CL). Full-length antibodies can be any class of antibodies, such as IgD, IgE, IgG, IgA, or IgM (or subclasses of the above), but antibodies do not need to belong to any particular class. Immunoglobulins can be assigned to different classes based on their antibody heavy chain constant region amino acid sequences. Typically, there are five major classes of immunoglobulins: IgA, IgD, IgE, IgG, and IgM, and several of these classes can be further divided into subclasses, such as IgG1, IgG2, IgG3, IgG4, IgA1, and IgA2.

As used herein, the term "specific binding" refers to a non-random binding reaction between two molecules, such as the binding of an antibody to an antigen epitope.

As used herein, the term "antigen-binding fragment" or "antigen-binding portion" refers to a portion or region of a complete antibody molecule that is responsible for binding to an antigen. The antigen-binding portion may comprise a heavy chain variable region (VH), a light chain variable region (VL), or both. Each of VH and VL typically comprises three complementary determining regions CDR1, CDR2, and CDR3.

It is well known to those skilled in the art that complementary determining regions (CDRs, typically CDR1, CDR2, and CDR3) are the regions in the variable regions that have the greatest impact on the affinity and specificity of the antibody. For a given antibody variable region amino acid sequence, the CDR amino acid sequences in the variable region amino acid sequence can be analyzed in a variety of ways. Herein, the CDR sequences of the heavy chain variable region and the light chain variable region are defined by the Kabat numbering system, in which "Kabat numbering system" refers to the EU index of Kabat et al. (see, for example, Kabat, Elvin A., et al. Sequences of Proteins of Immunological Interest. 5th ed, U.S. Dept. of Health and Human Services, Public Health Service, National Institutes of Health, 1991.).

As used herein, the term "isolated polynucleotide" refers to a polynucleotide that is (i) isolated from its natural environment, (ii) amplified by polymerase chain reaction, and/or (iii) synthesized in whole or in part, and refers to a single-stranded or double-stranded polymer of deoxyribonucleotide or ribonucleotide bases, and can include DNA and RNA molecules, both sense and antisense strands. The term includes cDNA, genomic DNA, mRNA, and recombinant DNA. An isolated polynucleotide can consist of an entire gene or a portion thereof.

As used herein, the term "vector" refers to a construct capable of delivering and preferably expressing one or more target genes or sequences in a host cell. Examples of vectors include, but are not limited to, viral vectors, naked DNA or RNA expression vectors, plasmids, cosmids or phage vectors, DNA or RNA expression vectors associated with cationic condensing agents, DNA or RNA expression vectors encapsulated in liposomes, and certain eukaryotic cells such as production cells.

As used herein, the term "host cell" refers to a cell containing a target protein of interest (e.g., the antibody or antigen-binding fragment thereof provided in any embodiment of the present disclosure). The target protein (e.g., the antibody or antigen-binding fragment thereof provided in any embodiment of the present disclosure) can be encoded by a polynucleotide, preferably an isolated polynucleotide. The polynucleotide can be present in the host cell in the following forms: (i) freely dispersed by itself, (ii) integrated into a recombinant vector, or (iii) integrated into a host cell genome or mitochondrial DNA. The host cell can be used to amplify and express a polynucleotide encoding a target protein of interest (e.g., the antibody or antigen-binding fragment thereof provided in any embodiment of the present disclosure). In a specific embodiment, the host cell can be selected from the group consisting of microbial host cells such as bacterial or yeast host cells, plant host cells, insect host cells or mammalian host cells, etc.

The term "detection label" used in the present disclosure refers to a label that can be detected directly or in certain conditions (e.g., providing illumination conditions, adding a color developing reagent, an enzyme, etc.). The detection label is directly or indirectly coupled to the antibody or antigen-binding fragment thereof of the present disclosure through a covalent bond or a non-covalent bond. The detection label theoretically has no effect or little effect on the protein structure, function, and activity of the antibody or antigen-binding fragment thereof. In some embodiments, the detection label has no effect on the function of the antibody or antigen-binding fragment thereof coupled thereto. Many such labels are readily known to those skilled in the art, for example, the detection label may be selected from the group consisting of radioactive labels (e.g., radioisotopes), fluorescent labels (e.g., fluorescein, phenanthridine dyes), chemiluminescent labels (e.g., acridinium ester compounds, cyanine dyes, luminol, isoluminol), biotin, colloidal gold, electrochemiluminescent labels (e.g., terpyridine ruthenium), quantum dots (e.g., gold quantum dots, CdSe quantum dots, ZnCdSe quantum dots, etc.) or enzymes (e.g., HRP (horse radish peroxidase), alkaline phosphatase, β-galactosidase).

The term "double antibody sandwich method" used in the present disclosure specifically refers to the enzyme-linked immunosorbent assay (ELISA) double antibody sandwich assay, which is an immunoassay technique commonly used in which an antigen is bound to two different antibodies. The antibodies separately bind to two different epitopes in the antigen that do not overlap or interfere. In this assay, a sandwich comprising a capture antibody, an antigen, and a detection antibody is formed, whereby the antigen bridges the two antibodies bound thereto. In some embodiments, the present disclosure provides an antibody pair, i.e., a combination of a capture antibody and a detection antibody, which can be further used to prepare a kit for detecting human immunodeficiency virus antigen.

It should be noted that the antibody or antigen-binding fragment thereof described in the present disclosure can be used in various assays for detecting human immunodeficiency virus antigens (e.g., HIV-1 p24 protein, HIV-2 p26 protein) in samples. Some suitable assays include, but are not limited to, protein blot analysis, radioimmunoassay, immunofluorescence assay, immunoprecipitation, equilibrium dialysis, immunodiffusion, electrochemiluminescence (ECL) immunoassay, immunohistochemistry, fluorescence activated cell sorting (FACS) or ELISA assays.

The present disclosure will now be further described by way of examples and specific embodiments and the advantages and various effects of the present disclosure will be more clearly presented. Those skilled in the art should understand that these specific embodiments and examples are used to illustrate the present disclosure, not to limit the present disclosure. As used herein, the same name is used to refer to the same antibody or the hybridoma cell line expressing it; the designation "rec" identifies recombinant antibodies. When the names of two antibodies differ only by the presence or absence of the "rec" designation, their heavy chain variable region sequences and light chain variable region sequences are identical.

### EXAMPLE 1

A mixture of the following three proteins was used as antigens: HIV-1 type M group p24 protein (abbreviated as p24_M), HIV-1 type O group p24 protein (abbreviated as p24_O), HIV-2 type p26 protein (abbreviated as p26). The sequences of the above three proteins could be found in the HIV Molecular Immunology Database-Los Alamos National Laboratory with the gene sequence accession numbers as follows: p24_M, AB034308; p24_O, AF383263; p26, AF025343. The above proteins were obtained by recombinant expression in *Escherichia coli* followed by purification.

The above antigens were used to immunize mice, rats and rabbits respectively, and the hybridoma cells obtained by immunization were screened to obtain hybridoma cells expressing anti-p24 and p26 antibodies. The antibodies expressed by these cells could specifically bind to both p24 protein (HIV-1 type M group and O group) and p26 protein (HIV-2 type). RNA was extracted from the screened hybridoma cells, and the sequences of the antibodies produced were sequenced. Some of the sequencing results are shown in Tables 1, 2 and 3.

**Table 1: CDR region sequences of antibodies obtained by mouse immunization**

| Name | Heavy chain complementary determining region | Amino acid sequence | SEQ ID NO: | Light chain complementary determining region | Amino acid sequence | SEQ ID NO: |
|---|---|---|---|---|---|---|
| p24_113 | HCDR1 | GYTFISYW | 1 | LCDR1 | RASGNIHNYLA | 4 |
| | HCDR2 | EIFPGSGHADYNEKFKD | 2 | LCDR2 | NAITLAD | 5 |
| | HCDR3 | STTRRIDY | 3 | LCDR3 | QHFWSTPLT | 6 |
| p24_138 | HCDR1 | GFAFSTYD | 7 | LCDR1 | RTSENIYSFLA | 10 |
| | HCDR2 | YISGGRGSTHYPDTVKG | 8 | LCDR2 | NVKTLAE | 11 |
| | HCDR3 | QRFSFGSSPFDV | 9 | LCDR3 | QHHYGTPWT | 12 |

**Table 2: CDR region sequences of antibodies obtained by rabbit immunization**

| Name | Heavy chain complementary determining region | Amino acid sequence | SEQ ID NO: | Light chain complementary determining region | Amino acid sequence | SEQ ID NO: |
|---|---|---|---|---|---|---|
| p24_131 | HCDR1 | GFSLSNYG | 13 | LCDR1 | QASESIDIWLV | 16 |
| | HCDR2 | YIGADGRIYYANWAK S | 14 | LCDR2 | DASKLAS | 17 |
| | HCDR3 | DMGTDAYGYATDI | 15 | LCDR3 | QQGYTYSDID NT | 18 |
| p24_140 | HCDR1 | GIDLSSNG | 19 | LCDR1 | QASESIDIWFS | 22 |
| | HCDR2 | FIKTTGNTYYASWAKG | 20 | LCDR2 | DASKLAA | 23 |
| | HCDR3 | DPDYSYGYVLDP | 21 | LCDR3 | QQGYSVSDVD NI | 24 |
| p24_151 | HCDR1 | GFTISSSYY | 25 | LCDR1 | QASQNIYNNL A | 28 |
| | HCDR2 | CIYGGSSGSTSYASWA KG | 26 | LCDR2 | RASTLES | 29 |
| | HCDR3 | DRGDWYVDL | 27 | LCDR3 | QGYAYSITTD YI | 30 |
| p24_179 | HCDR1 | GFDFSGVG | 31 | LCDR1 | QASQSIDSYLS | 34 |
| | HCDR2 | YIYPKFGVRNYANSVK G | 32 | LCDR2 | GASTLES | 35 |
| | HCDR3 | AGYPGYGYYFDL | 33 | LCDR3 | QGGYYSNSVI GA | 36 |

**Table 3: CDR region sequences of antibodies obtained by rat immunization**

| Name | Heavy chain complementary determining region | Amino acid sequence | SEQ ID NO: | Light chain complementary determining region | Amino acid sequence | SEQ ID NO: |
|---|---|---|---|---|---|---|
| p24_252 | HCDR1 | GFSLTSYN | 37 | LCDR1 | RASETVTTGVH | 40 |
| | HCDR2 | IVWSGGLIDYNSTLKS | 38 | LCDR2 | GASNLES | 41 |
| | HCDR3 | ENRHSHHDYFDY | 39 | LCDR3 | QQTWNDPLT | 42 |

### EXAMPLE 2

Antibodies were prepared according to the following methods:
1. Monoclonal antibodies p24_113 and p24_138:
   The hybridoma cells p24_113 and p24_138 obtained in Example 1 were injected intraperitoneally into mice for the production of monoclonal antibodies in ascites, followed by purification, thereby preparing the monoclonal antibodies p24_113 and p24_138.
2. Recombinant antibodies P24_rec_131, p24_rec_140, p24_rec_151, p24_rec_179, and p24_rec_252:
   For the hybridoma cells P24_131, p24_140, p24_151, and p24_179 obtained in Example 1, the heavy chain variable region sequences and light chain variable region sequences of the antibodies expressed by the above hybridoma cells were obtained according to the antibody sequencing results. The heavy chain variable region sequence obtained by sequencing was fused with the heavy chain constant region of rabbit immunoglobulin IgG isotype to obtain the heavy chain sequence of the recombinant antibody; the light chain variable region sequence obtained by sequencing was fused with the light chain constant region of rabbit immunoglobulin IgG isotype to obtain the light chain sequence of the recombinant antibody. Thus, the heavy chain sequence and light chain sequence of the recombinant antibody were obtained respectively, the expression vectors expressing them were constructed, and they were expressed and purified by the mammalian cell line Expi293F.

For the hybridoma cell p24_252 obtained in Example 1, the heavy chain variable region sequence and light chain variable region sequence of the antibody expressed by the above hybridoma cell were obtained according to the antibody sequencing results. The heavy chain variable region sequence obtained by sequencing was fused with the heavy chain constant region of sheep immunoglobulin IgG isotype to obtain the heavy chain sequence of the recombinant antibody; the light chain variable region sequence obtained by sequencing was fused with the light chain constant region of sheep immunoglobulin IgG isotype to obtain the light chain sequence of the recombinant antibody. Thus, the heavy chain sequence and light chain sequence of the recombinant antibody were obtained respectively, the expression vectors for expressing them were constructed, and they were expressed and purified by the mammalian cell line Expi293F.

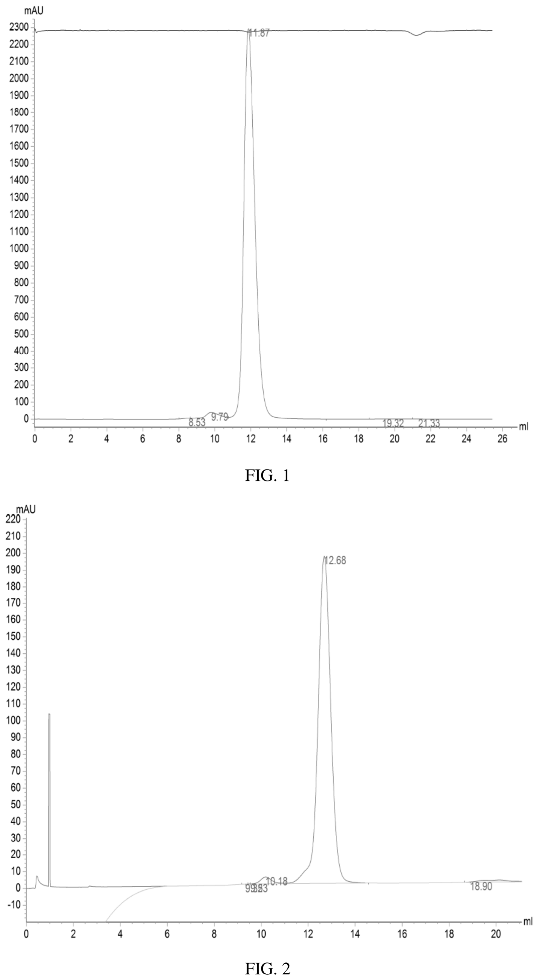

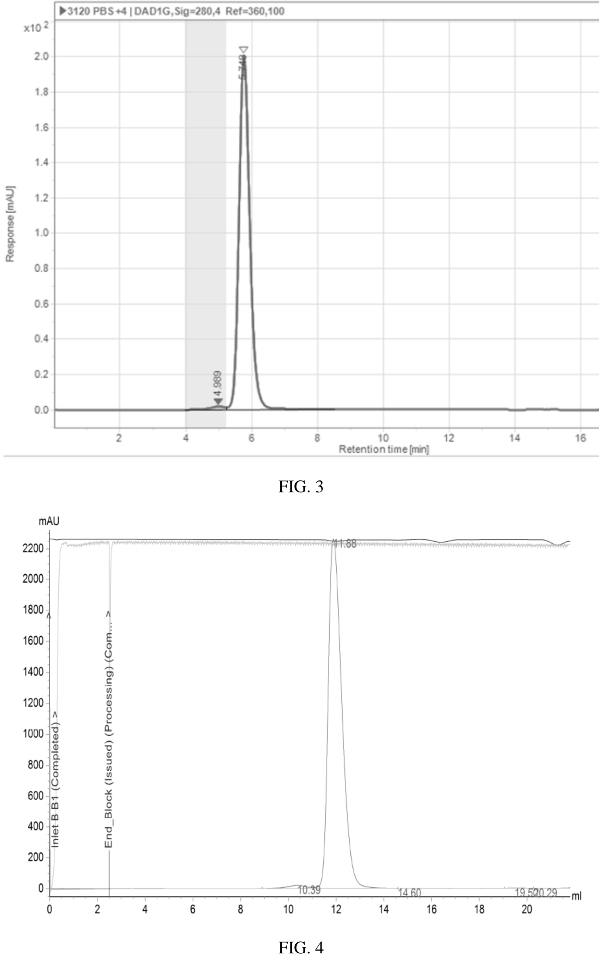

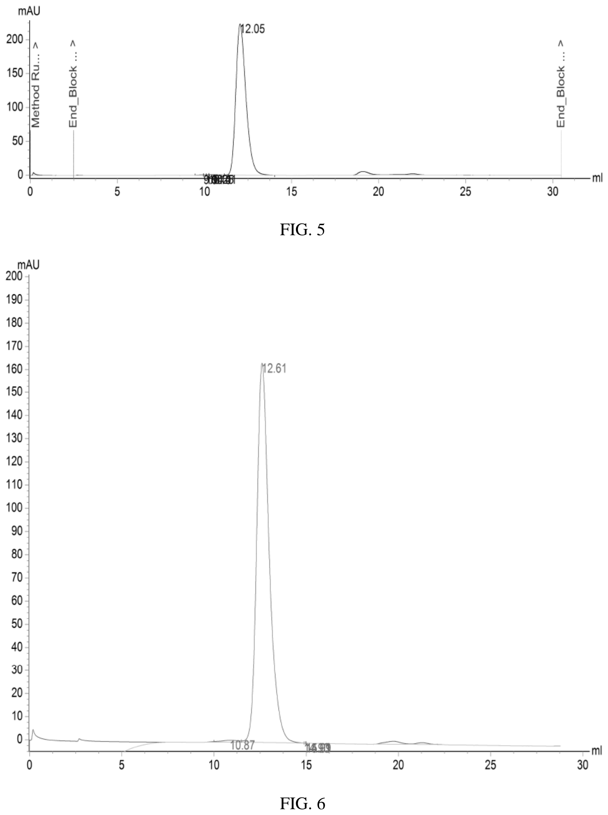

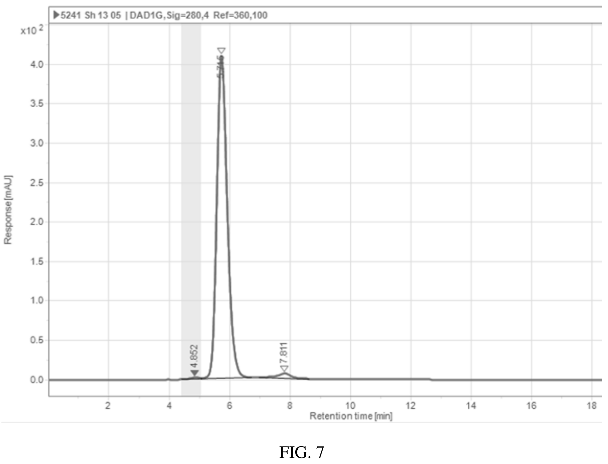

The antibodies prepared above were subjected to size exclusion (SEC) chromatography analysis, and the results are shown in Figures 1 to 7. The purified antibodies show high purity.

The following biological materials are all deposited in the Russian National Collection of Industrial Microorganisms (VKPM) on September 17, 2024, with the deposit details as follows.

The hybridoma cell line with the deposit number of VKPM H-227 is the hybridoma cell line named p24_138 (The hybridoma of p24_138); and used to produce monoclonal antibody p24_138.

The hybridoma cell line with the deposit number of VKPM H-228 is the hybridoma cell line named p24_113 (The hybridoma of p24_113); and used to produce monoclonal antibody p24_113.

The *Escherichia coli* with the deposit number of VKPM B-15024 is named *Escherichia coli* Rosetta^{™} (DE3) pLysS p24_rec_131 VH; and used to produce a plasmid encoding the p24_rec_131 heavy chain sequence.

The *Escherichia coli* with the deposit number of VKPM B-15025 is named *Escherichia coli* Rosetta^{™} (DE3) pLysS p24_rec_131 VL; and used to produce a plasmid encoding the p24_rec_131 light chain sequence.

The *Escherichia coli* with the deposit number of VKPM B-15026 is named *Escherichia coli* Rosetta^{™} (DE3) pLysS p24_rec_140 VH; and used to produce a plasmid encoding the p24_rec_140 heavy chain sequence.

The *Escherichia coli* with the deposit number of VKPM B-15027 is named *Escherichia coli* Rosetta^{™} (DE3) pLysS p24_rec_140 VL; and used to produce a plasmid encoding the p24_rec_140 light chain sequence.

The *Escherichia coli* with the deposit number of VKPM B-15028 is named *Escherichia coli* Rosetta^{™} (DE3) pLysS p24_rec_151 VH; and used to produce a plasmid encoding the p24_rec_151 heavy chain sequence.

The *Escherichia coli* with the deposit number of VKPM B-15029 is named *Escherichia coli* Rosetta^{™} (DE3) pLysS p24_rec_151 VL; and used to produce a plasmid encoding the p24_rec_151 light chain sequence.

The *Escherichia coli* with the deposit number of VKPM B-15030 is named *Escherichia coli* Rosetta^{™} (DE3) pLysS p24_rec_179 VH; and used to produce a plasmid encoding the p24_rec_179 heavy chain sequence.

The *Escherichia coli* with the deposit number of VKPM B-15031 is named *Escherichia coli* Rosetta^{™} (DE3) pLysS p24_rec_179 VL; and used to produce a plasmid encoding the p24_rec_179 light chain sequence.

The *Escherichia coli* with the deposit number of VKPM B-15032 is named *Escherichia coli* Rosetta^{™} (DE3) pLysS p24_rec_252 VH; and used to produce a plasmid encoding the p24_rec_252 heavy chain sequence.

The *Escherichia coli* with the deposit number of VKPM B-15033 is named *Escherichia coli* Rosetta^{™} (DE3) pLysS p24_rec_252 VL; and used to produce a plasmid encoding the p24_rec_252 light chain sequence.

### EXAMPLE 3

In this example, the affinity of the antibodies for HIV-1 type M group p24 protein was measured by a method comprising the following steps:
The antibodies prepared in Example 2 were biotin-labeled to obtain biotinylated antibodies. Each of the biotinylated antibodies was added to a buffer (50 mM Tris-HCl buffer, pH 7.5, 150 mM NaCl, 0.01% Tween 40, 0.5% BSA, and 0.05% NaN₃) so that the final concentration of the biotinylated antibody was 10 µg/ml to prepare an antibody solution to be tested. The antibody solution to be tested was added to streptavidin biosensors (FORTEBIO SA (streptavidin) Dip and Read biosensors) and kept for 10 minutes, followed by washing with the above buffer for 10 minutes. p24 protein solutions with concentrations of 20nM, 10nM, and 5nM were added respectively, and incubated for 20 minutes. Then, the dissociation was carried out for 30 minutes and the test results of the sensors were read, as shown in Table 4:

**Table 4**

| Antibody | Ka (1/Ms) | Kd (1/s) | KD (M) |
|---|---|---|---|
| p24_113 | 4.92E+05 | 5.28E-05 | 1.08E-10 |
| p24_138 | 2.91E+05 | 7.11E-05 | 2.42E-10 |
| p24_rec_179 | 3.07E+05 | 1.28E-04 | 4.19E-10 |
| p24_rec_151 | 2.69E+05 | 4.12E-05 | 1.53E-10 |
| p24_rec_140 | 4.04E+05 | 1.70E-04 | 4.23E-10 |
| p24_rec_131 | 3.42E+05 | 7.80E-05 | 2.29E-10 |
| p24_rec_252 | 8.25E+05 | 1.34E-04 | 1.63E-10 |

As shown in Table 4, the above antibodies show good affinity for p24 protein.

### EXAMPLE 4

When using the double antibody sandwich method for immunoassay, two antibodies (or antibody pair) should be used simultaneously, one of which is a capture antibody and the other is a detection antibody. In this example, the antibodies prepared in Example 2 were combined in pairs, and then antibody pairs suitable for detecting HIV-1 and HIV-2 by the double antibody sandwich method were screened, comprising the following steps:
Preparation of capture antibody reagent: The capture antibody was labeled with biotin, the biotin-labeled capture antibody was incubated with streptavidin-labeled magnetic beads (Dynabeads^{™} MyOne^{™} Streptavidin T1) to obtain magnetic beads coupled with the capture antibody, and they were added to buffer A (50 mM Tris-HCl, pH 7.4, 150 mM NaCl, 2% BSA, 0.2% Tween-20, 0.05% ProClin 300, 0.09% NaN₃) at a final concentration of 0.2 mg/ml to prepare a capture antibody reagent.

Detection antibody reagent: The detection antibody was labeled with alkaline phosphatase, and then added to buffer B (50 mM MES, pH6, 0.9% NaCl, 1% BSA, 0.05% Tween-20, 0.048% ProClin 300, 2 mM MgCl₂, 0.1 mM ZnCl₂) at a final concentration of 0.5 µg/ml to prepare a detection antibody reagent.

Antigens to be tested: The HIV-1 type p24 antigen of World Health Organization (WHO) international standard (purchased from NIBSC, Cat. No.: 90/636), HIV-1 type M group p24 protein in Example 1 (abbreviated as p24_M in Table 5), HIV-1 type O group p24 protein (abbreviated as p24_O in Table 5), HIV-2 type p26 protein (abbreviated as p26 in Table 5) were used as antigens respectively, and subjected to gradient dilution with the above buffer B to prepare antigens to be tested.

Immunoassay steps: Mindray's fully automatic chemiluminescence immunoassay analyzer CL-6000i was used, and the test was performed according to the instructions using the substrate solution provided with the instrument. Chemiluminescence is expressed in relative light units (RLU).

The antigens to be tested were detected by the above method, and the minimum detection limit was detected. At the same time, a positive control using the Abbott commercially available kit was set. The limit of detection (LOD) is described as follows: at least 5 gradient dilution concentrations were set near the LOD for each antigen sample to be tested; 20 replicates were set for each antigen sample to be tested at each concentration value. LOD was calculated according to the CLSI (Clinical and Laboratory Standards Institute) EP17-A document, and the beta error risk = 0.05.

Some of the antibody pairs screened and their LOD values are shown in Table 5.

**Table 5**

| No. | Capture antibody | Detection antibody | LOD | | | |
|---|---|---|---|---|---|---|
| | | | WHO international standard HIV-1 type p24 antigen, IU/ml | p24_M, pg/ml | p24_O, pg/ml | p26, pg/ml |
| 1 | p24_138 | p24_rec_252 | 0.64 | 4 | 1.5 | 15 |
| 2 | p24_138 | p24_113 | 0.64 | 2 | 2 | 8 |
| 3 | p24_138 | p24_rec_179 | 0.55 | 1.5 | 1.5 | 8 |
| 4 | p24_rec_151 | p24_rec_252 | 0.5 | 2 | 1.5 | 20 |
| 5 | p24_rec_179 | p24_rec_252 | 0.55 | 4 | 1.5 | 20 |
| 6 | p24_138 | p24_rec_151 | 0.64 | 4 | 2 | 15 |
| 7 | p24_rec_140 | p24_rec_252 | 0.64 | 4 | 4 | 25 |
| 8 | p24_138 | p24_rec_131 | 0.64 | 2 | 2 | 8 |
| 9 | p24_rec_131 | p24_rec_252 | 0.55 | 4 | 4 | 30 |
| 10 | p24_138 | p24_rec_140 | 0.64 | 2 | 2 | 8 |
| 11 | p24_rec_151 | p24_rec_140 | 0.55 | 2 | 4 | 5 |
| 12 | p24_rec_151 | p24_rec_179 | 0.5 | 1 | 1 | 3 |
| 13 | p24_rec_179 | p24_113 | 0.55 | 2 | 1.5 | 8 |
| 14 | p24_113 | p24_rec_151 | 0.55 | 2 | 2 | 8 |
| 15 | p24_113 | p24_rec_131 | 0.64 | 4 | 4 | 4 |
| 16 | p24_113 | p24_rec_140 | 0.64 | 4 | 4 | 4 |
| 17 | p24_rec_151 | p24_rec_131 | 0.55 | 2 | 4 | 8 |
| 18 | Abbott HIV Ag/Ab Combo Kit (Cat. No. 4J2784) | | 1.00 | 5 | 4 | Not detected when p26 was 1 ng/ml |

The above antibody pairs could simultaneously detect HIV-1 type O group and M group p24 proteins, HIV-2 type p26 protein, and had been verified to detect WHO international standard HIV-1 type p24 antigen. Moreover, the limits of detection of the above-mentioned antibody pairs for the above-mentioned antigens were better than those of Abbott HIV Ag/Ab combo kit that had been on the market.

The above is only preferred specific embodiments of the present disclosure, but the protection scope of the present disclosure is not limited thereto. Any changes or substitutions that can be easily conceived by those skilled in the art within the technical scope of the disclosure should fall within the protection scope of the present disclosure. Therefore, the protection scope of the present disclosure shall be based on the protection scope of the claims.

## Claims

1. An antibody or antigen-binding fragment thereof, wherein the antibody or antigen-binding fragment thereof is capable of specifically binding to human immunodeficiency virus type 1 p24 protein and human immunodeficiency virus type 2 p26 protein; the antibody or antigen-binding fragment thereof comprises a heavy chain variable region comprising HCDR1, HCDR2 and HCDR3 amino acid sequences and a light chain variable region comprising LCDR1, LCDR2 and LCDR3 amino acid sequences;
wherein, the amino acid sequence of HCDR1 is selected from SEQ ID NOs: 1, 7, 13, 19, 25, 31, 37 or variants thereof; the amino acid sequence of HCDR2 is selected from SEQ ID NOs: 2, 8, 14, 20, 26, 32, 38 or variants thereof; the amino acid sequence of HCDR3 is selected from SEQ ID NOs: 3, 9, 15, 21, 27, 33, 39 or variants thereof;
the amino acid sequence of LCDR1 is selected from SEQ ID NOs: 4, 10, 16, 22, 28, 34, 40 or variants thereof; the amino acid sequence of LCDR2 is selected from SEQ ID NOs: 5, 11, 17, 23, 29, 35, 41 or variants thereof; the amino acid sequence of the LCDR3 is selected from SEQ ID NOs: 6, 12, 18, 24, 30, 36, 42 or variants thereof;
wherein the variant comprises an amino acid mutation as compared with the amino acid sequence from which it is derived, and the amino acid mutation is a substitution, deletion or addition of one or several amino acids; the variant has an identity of at least 80%, at least 85%, at least 90%, at least 91%, at least 92%, at least 93%, at least 94%, at least 95%, at least 96%, at least 97%, at least 98% or at least 99% as compared with the amino acid sequence from which it is derived.

2. The antibody or antigen-binding fragment thereof according to claim 1, wherein:
the amino acid sequence of HCDR1 is SEQ ID NO: 1, the amino acid sequence of HCDR2 is SEQ ID NO: 2, and the amino acid sequence of HCDR3 is SEQ ID NO: 3; the amino acid sequence of LCDR1 is SEQ ID NO: 4, the amino acid sequence of LCDR2 is SEQ ID NO: 5, and the amino acid sequence of LCDR3 is SEQ ID NO: 6; or,
the amino acid sequence of HCDR1 is SEQ ID NO: 7, the amino acid sequence of HCDR2 is SEQ ID NO: 8, and the amino acid sequence of HCDR3 is SEQ ID NO: 9; the amino acid sequence of LCDR1 is SEQ ID NO: 10, the amino acid sequence of LCDR2 is SEQ ID NO: 11, and the amino acid sequence of LCDR3 is SEQ ID NO: 12; or,
the amino acid sequence of HCDR1 is SEQ ID NO: 13, the amino acid sequence of HCDR2 is SEQ ID NO: 14, and the amino acid sequence of HCDR3 is SEQ ID NO: 15; the amino acid sequence of LCDR1 is SEQ ID NO: 16, the amino acid sequence of LCDR2 is SEQ ID NO: 17, and the amino acid sequence of LCDR3 is SEQ ID NO: 18; or,
the amino acid sequence of HCDR1 is SEQ ID NO: 19, the amino acid sequence of HCDR2 is SEQ ID NO: 20, and the amino acid sequence of HCDR3 is SEQ ID NO: 21; the amino acid sequence of LCDR1 is SEQ ID NO: 22, the amino acid sequence of LCDR2 is SEQ ID NO: 23, and the amino acid sequence of LCDR3 is SEQ ID NO: 24; or,
the amino acid sequence of HCDR1 is SEQ ID NO: 25, the amino acid sequence of HCDR2 is SEQ ID NO: 26, and the amino acid sequence of HCDR3 is SEQ ID NO: 27; the amino acid sequence of LCDR1 is SEQ ID NO: 28, the amino acid sequence of LCDR2 is SEQ ID NO: 29, and the amino acid sequence of LCDR3 is SEQ ID NO: 30; or,
the amino acid sequence of HCDR1 is SEQ ID NO: 31, the amino acid sequence of HCDR2 is SEQ ID NO: 32, and the amino acid sequence of HCDR3 is SEQ ID NO: 33; the amino acid sequence of LCDR1 is SEQ ID NO: 34, the amino acid sequence of LCDR2 is SEQ ID NO: 35, and the amino acid sequence of LCDR3 is SEQ ID NO: 36; or,
the amino acid sequence of HCDR1 is SEQ ID NO: 37, the amino acid sequence of HCDR2 is SEQ ID NO: 38, and the amino acid sequence of HCDR3 is SEQ ID NO: 39; the amino acid sequence of LCDR1 is SEQ ID NO: 40, the amino acid sequence of LCDR2 is SEQ ID NO: 41, and the amino acid sequence of LCDR3 is SEQ ID NO: 42.

3. The antibody or antigen-binding fragment thereof according to claim 1 or 2, wherein the antibody is a full-length antibody, a Fab fragment, a Fab' fragment, a F(ab')₂ fragment, a double-chain Fv fragment, or a single-chain Fv fragment;
preferably, the type of the antibody or antigen-binding fragment thereof is IgG, IgM, IgE, IgD or IgA;
preferably, the antibody or antigen-binding fragment thereof is a mouse antibody, a rabbit antibody or a humanized antibody.

4. The antibody or antigen-binding fragment thereof according to any one of claims 1 to 3, wherein the antibody comprises a constant region;
preferably, the constant region comprises a heavy chain constant region and/or a light chain constant region;
preferably, the constant region comprises a mouse constant region, a rat constant region, a rabbit constant region, a sheep constant region, a monkey constant region or a human constant region;
preferably, the heavy chain constant region is a heavy chain constant region of IgG, e.g., IgG1, IgG2, IgG3 or IgG4;
preferably, the light chain constant region is a κ or λ type light chain constant region.

5. The antibody or antigen-binding fragment thereof according to any one of claims 1 to 4, wherein the antibody or antigen-binding fragment thereof is coupled with a detection label; the detection label comprises at least one selected from the group consisting of radioactive label, fluorescent label, chemiluminescent label, biotin, colloidal gold, electrochemiluminescent label, quantum dot or enzyme.

6. An antibody or antigen-binding fragment thereof, wherein:
the antibody is capable of being produced by a hybridoma cell line deposited in VKPM with a deposit number of VKPM H-227; or,
the antibody is capable of being produced by a hybridoma cell line deposited in VKPM with a deposit number of VKPM H-228; or,
the antibody is capable of being produced by a plasmid in *Escherichia coli* deposited in VKPM with a deposit number of VKPM B-15024 and a plasmid in *Escherichia coli* deposited in VKPM with a deposit number of VKPM B-15025; or,
the antibody is capable of being produced by a plasmid in *Escherichia coli* deposited in VKPM with a deposit number of VKPM B-15026 and a plasmid in *Escherichia coli* deposited in VKPM with a deposit number of VKPM B-15027; or,
the antibody is capable of being produced by a plasmid in *Escherichia coli* deposited in VKPM with a deposit number of VKPM B-15026 and a plasmid in *Escherichia coli* deposited in VKPM with a deposit number of VKPM B-15027; or,
the antibody is capable of being produced by a plasmid in *Escherichia coli* deposited in VKPM with a deposit number of VKPM B-15028 and a plasmid in *Escherichia coli* deposited in VKPM with a deposit number of VKPM B-15029; or,
the antibody is capable of being produced by a plasmid in *Escherichia coli* deposited in VKPM with a deposit number of VKPM B-15030 and a plasmid in *Escherichia coli* deposited in VKPM with a deposit number of VKPM B-15031; or,
the antibody is capable of being produced by a plasmid in *Escherichia coli* deposited in VKPM with a deposit number of VKPM B-15032 and a plasmid in *Escherichia coli* deposited in VKPM with a deposit number of VKPM B-15033.

7. An isolated polynucleotide, encoding the antibody or antigen-binding fragment thereof according to any one of claims 1 to 6.

8. A vector, comprising the isolated polynucleotide according to claim 7.

9. A host cell, comprising: the isolated polynucleotide according to claim 7, or the vector according to claim 8.

10. An antibody pair, comprising a capture antibody and a detection antibody, wherein at least one of the capture antibody and the detection antibody is selected from the antibody or antigen-binding fragment thereof according to any one of claims 1 to 6;
preferably, the detection antibody bears a detection label, e.g., radioactive label, fluorescent label, chemiluminescent label, biotin, colloidal gold, electrochemiluminescent label, quantum dot or enzyme;
preferably, the capture antibody is coated on the surface of a solid carrier, e.g., magnetic bead or microtiter plate.

11. The antibody pair according to claim 10, wherein the capture antibody and the detection antibody are each independently selected from the antibody or antigen-binding fragment thereof according to any one of claims 1 to 6; and the capture antibody and the detection antibody are different;
preferably, the antibody pair is selected from any one of the following groups (i) to (vi):
(i) a capture antibody comprising a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, and a LCDR3 with an amino acid sequence as set forth in SEQ ID NO: 6; and,
a detection antibody, which is one selected from the following group:
the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, and a LCDR3 with an amino acid sequence as set forth in SEQ ID NO: 18;
the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, and a LCDR3 with an amino acid sequence as set forth in SEQ ID NO: 24; or,
the detection antibody comprising a HCDR1 with an amino acid sequence as set forth in SEQ ID NO: 25, a HCDR2 with an amino acid sequence as set forth in SEQ ID NO: 26, a HCDR3 with an amino acid sequence as set forth in SEQ ID NO: 27, a LCDR1 with an amino acid sequence as set forth in SEQ ID NO: 28, a LCDR2 with an amino acid sequence as set forth in SEQ ID NO: 29, and a LCDR3 with an amino acid sequence as set forth in SEQ ID NO: 30;
(ii) a capture antibody comprising a HCDR1 with an amino acid sequence as set forth in SEQ ID NO: 7, a HCDR2 with an amino acid sequence as set forth in SEQ ID NO: 8, a HCDR3 with an amino acid sequence as set forth in SEQ ID NO: 9, a LCDR1 with an amino acid sequence as set forth in SEQ ID NO: 10, a LCDR2 with an amino acid sequence as set forth in SEQ ID NO: 11, and a LCDR3 with an amino acid sequence as set forth in SEQ ID NO: 12; and,
a detection antibody, which is one selected from the following group:
the detection antibody comprising a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, and a LCDR3 with an amino acid sequence as set forth in SEQ ID NO: 6;
the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, a LCDR3 with an amino acid sequence as set forth in SEQ ID NO: 18;
the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, LCDR3 with an amino acid sequence as set forth in SEQ ID NO: 24;
the detection antibody comprising a HCDR1 with an amino acid sequence as set forth in SEQ ID NO: 25, a HCDR2 with an amino acid sequence as set forth in SEQ ID NO: 26, a HCDR3 with an amino acid sequence as set forth in SEQ ID NO: 27, a LCDR1 with an amino acid sequence as set forth in SEQ ID NO: 28, a LCDR2 with an amino acid sequence as set forth in SEQ ID NO: 29, and a LCDR3 with an amino acid sequence as set forth in SEQ ID NO: 30;
the detection antibody comprising a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33, a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; or,
the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42;
(iii) a capture antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, a LCDR3 with an amino acid sequence as set forth in SEQ ID NO: 18; and,
a detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, a LCDR3 with an amino acid sequence as set forth in SEQ ID NO: 42;
(iv) a capture antibody, comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, a LCDR3 with an amino acid sequence as set forth in SEQ ID NO: 24; and,
a detection antibody, comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, a LCDR3 with an amino acid sequence as set forth in SEQ ID NO: 42;
(v) a capture antibody comprising a HCDR1 with an amino acid sequence as set forth in SEQ ID NO: 25, a HCDR2 with an amino acid sequence as set forth in SEQ ID NO: 26, a HCDR3 with an amino acid sequence as set forth in SEQ ID NO: 27; a LCDR1 with an amino acid sequence as set forth in SEQ ID NO: 28, a LCDR2 with an amino acid sequence as set forth in SEQ ID NO: 29, and a LCDR3 with an amino acid sequence as set forth in SEQ ID NO: 30; and,
a detection antibody, which is one selected from the following group:
the detection antibody comprising a HCDR1 with an amino acid sequence as set forth in SEQ ID NO: 13, a HCDR2 with an amino acid sequence as set forth in SEQ ID NO: 14, a HCDR3 with an amino acid sequence as set forth in SEQ ID NO: 15, a LCDR1 with an amino acid sequence as set forth in SEQ ID NO: 16, a LCDR2 with an amino acid sequence as set forth in SEQ ID NO: 17, and a LCDR3 with an amino acid sequence as set forth in SEQ ID NO: 18;
the detection antibody comprising a HCDR1 with an amino acid sequence as set forth in SEQ ID NO: 19, a HCDR2 with an amino acid sequence as set forth in SEQ ID NO: 20, a HCDR3 with an amino acid sequence as set forth in SEQ ID NO: 21, a LCDR1 with an amino acid sequence as set forth in SEQ ID NO: 22, a LCDR2 with an amino acid sequence as set forth in SEQ ID NO: 23, a LCDR3 with an amino acid sequence as set forth in SEQ ID NO: 24;
the detection antibody comprising a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33, a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; or,
the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, a LCDR3 with an amino acid sequence as set forth in SEQ ID NO: 42;
(vi) a capture antibody comprising a HCDR1 with an amino acid sequence as set forth in SEQ ID NO: 31, a HCDR2 with an amino acid sequence as set forth in SEQ ID NO: 32, a HCDR3 with an amino acid sequence as set forth in SEQ ID NO: 33; a LCDR1 with an amino acid sequence as set forth in SEQ ID NO: 34, a LCDR2 with an amino acid sequence as set forth in SEQ ID NO: 35, and a LCDR3 with an amino acid sequence as set forth in SEQ ID NO: 36; and,
a detection antibody, which is one selected from the following group:
the detection antibody comprising a HCDR1 with an amino acid sequence as set forth in SEQ ID NO: 1, a HCDR2 with an amino acid sequence as set forth in SEQ ID NO: 2, a HCDR3 with an amino acid sequence as set forth in SEQ ID NO: 3, a LCDR1 with an amino acid sequence as set forth in SEQ ID NO: 4, a LCDR2 with an amino acid sequence as set forth in SEQ ID NO: 5, a LCDR3 with an amino acid sequence as set forth in SEQ ID NO: 6; or,
the detection antibody comprising a HCDR1 with an amino acid sequence as set forth in SEQ ID NO: 37, a HCDR2 with an amino acid sequence as set forth in SEQ ID NO: 38, a HCDR3 with an amino acid sequence as set forth in SEQ ID NO: 39, a LCDR1 with an amino acid sequence as set forth in SEQ ID NO: 40, a LCDR2 with an amino acid sequence as set forth in SEQ ID NO: 41, and a LCDR3 with an amino acid sequence as set forth in SEQ ID NO: 42.

12. The antibody pair according to claim 10, wherein the antibody pair is selected from any one of the following groups (a) to (f):
(a) a capture antibody, which is an antibody produced by a hybridoma cell line deposited in VKPM with a deposit number of VKPM H-228; and,
a detection antibody, which is one selected from the following group:
the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025;
the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027; or
the detection antibody is an antibody produced by a plasmid in *Escherichia coli* deposited in VKPM with a deposit number of VKPM B-15028 and a plasmid in *Escherichia coli* deposited in VKPM with a deposit number of VKPM B-15029;
(b) a capture antibody, which is an antibody produced by a hybridoma cell line deposited in VKPM with a deposit number of VKPM H-227; and,
a detection antibody, which is one selected from the following group:
the detection antibody is an antibody produced by a hybridoma cell line deposited in VKPM with a deposit number of VKPM H-228;
the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025;
the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027;
the detection antibody is an antibody produced by a plasmid in *Escherichia coli* deposited in VKPM with a deposit number of VKPM B-15028 and a plasmid in *Escherichia coli* deposited in VKPM with a deposit number of VKPM B-15029;
the detection antibody is an antibody produced by a plasmid in *Escherichia coli* deposited in VKPM with a deposit number of VKPM B-15030 and a plasmid in *Escherichia coli* deposited in VKPM with a deposit number of VKPM B-15031; or,
the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033;
(c) a capture antibody, which is an antibody produced by a plasmid in *Escherichia coli* deposited in VKPM with a deposit number of VKPM B-15024 and a plasmid in *Escherichia coli* deposited in VKPM with a deposit number of VKPM B-15025; and,
a detection antibody, which is an antibody produced by a plasmid in *Escherichia coli* deposited in VKPM with a deposit number of VKPM B-15032 and a plasmid in *Escherichia coli* deposited in VKPM with a deposit number of VKPM B-15033;
(d) a capture antibody, which is an antibody produced by a plasmid in *Escherichia coli* deposited in VKPM with a deposit number of VKPM B-15026 and a plasmid in *Escherichia coli* deposited in VKPM with a deposit number of VKPM B-15027; and,
a detection antibody, which is an antibody produced by a plasmid in *Escherichia coli* deposited in VKPM with a deposit number of VKPM B-15032 and a plasmid in *Escherichia coli* deposited in VKPM with a deposit number of VKPM B-15033;
(e) a capture antibody, which is an antibody produced by a plasmid in *Escherichia coli* deposited in VKPM with a deposit number of VKPM B-15028 and a plasmid in *Escherichia coli* deposited with a deposit number of VKPM B-15029; and,
a detection antibody, which is one selected from the following group:
the detection antibody is an antibody produced by a plasmid in *Escherichia coli* deposited in VKPM with a deposit number of VKPM B-15024 and a plasmid in *Escherichia coli* deposited in VKPM with a deposit number of VKPM B-15025;
the detection antibody is an antibody produced by a plasmid in *Escherichia coli* deposited in VKPM with a deposit number of VKPM B-15026 and a plasmid in *Escherichia coli* deposited in VKPM with a deposit number of VKPM B-15027;
the detection antibody is an antibody produced by a plasmid in *Escherichia coli* deposited in VKPM with a deposit number of VKPM B-15030 and a plasmid in *Escherichia coli* deposited in VKPM with a deposit number of VKPM B-15031; or,
the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033;
(f) a capture antibody, which is an antibody produced by a plasmid in *Escherichia coli* deposited in VKPM with a deposit number of VKPM B-15030 and a plasmid in *Escherichia coli* deposited in VKPM with a deposit number of VKPM B-15031; and,
a detection antibody, which is one selected from the following group:
the detection antibody is an antibody produced by a hybridoma cell line deposited in VKPM with a deposit number of VKPM H-228; or,
the detection antibody is an antibody produced by a plasmid in *Escherichia coli* deposited in VKPM with a deposit number of VKPM B-15032 and a plasmid in *Escherichia coli* deposited in VKPM with a deposit number of VKPM B-15033.

13. A kit, comprising: the antibody or antigen-binding fragment thereof according to any one of claims 1 to 6, or the antibody pair according to any one of claims 10 to 12.

14. A method for detecting presence and/or amount of human immunodeficiency virus or an antigen thereof in a sample, comprising: using the antibody or antigen-binding fragment thereof according to any one of claims 1 to 6 or the antibody pair according to any one of claims 10 to 12 to perform immunoassay on the sample;
preferably, the method comprises:
(i) contacting the sample with the capture antibody of the antibody pair to form an antibody-antigen complex;
(ii) contacting the antibody-antigen complex with the detection antibody of the antibody pair to form an antibody-antigen-antibody complex; and
(iii) detecting the antibody-antigen-antibody complex, preferably wherein presence of the complex indicates presence of human immunodeficiency virus or an antigen thereof in the sample;
preferably, the human immunodeficiency virus comprises human immunodeficiency virus type 1 and human immunodeficiency virus type 2; preferably, the antigen is selected from HIV-1 antigen (e.g., p24) and HIV-2 antigen (e.g., p26);
preferably, the immunoassay is selected from enzyme immunoassay or chemiluminescence immunoassay.

15. A method for diagnosing or aiding in diagnosis of acquired immunodeficiency syndrome or AIDS-related syndrome, comprising: detecting the presence of human immunodeficiency virus or an antigen thereof in a sample from the subject by the method of claim 14; preferably wherein presence of human immunodeficiency virus or an antigen thereof indicates that the subject has sustained or may have sustained acquired immunodeficiency syndrome.
